# EUROPÄISCHE PATENTSCHRIFT

(11) **EP 1 162 947 B1**
(45) Veröffentlichungstag und Bekanntmachung des Hinweises auf die Patenterteilung: **02.11.2005**
(21) Anmeldenummer: 00929212.9
(22) Anmeldetag: 22.03.2000
(51) Int. Cl.: A61K 9/08

(54) **POTENTIERUNGSMEDIUM**
POTENTIATION MEDIUM
AGENT DE POTENTIALISATION

(30) Priorität: 22.03.1999 DE 19912933
(43) Veröffentlichungstag der Anmeldung: 19.12.2001
(73) Patentinhaber: Kwalis Qualitätsforschung Fulda GmbH, 36160 Dipperz (DE)
(72) Erfinder: STRUBE, Jürgen, D-36157 Ebersburg (DE); STOLZ, Peter, D-36157 Ebersburg (DE); MAIER, Walter, D-91560 Weissenbronn (DE); GUTBERLET, Wolfgang, D-36160 Dipperz (DE)
(74) Vertreter: Hebing, Norbert
(86) Internationale Anmeldenummer: PCT/DE2000/000817
(87) Internationale Veröffentlichungsnummer: WO 2000/056284

(56) Entgegenhaltungen:
- EP-A- 0 818 203
- FR-A- 2 659 553

## Beschreibung

Die Erfindung betrifft ein Verfahren zur Herstellung eines potenzierten Arzneimittels für die Anwendung bei Menschen, Tieren oder Pflanzen.

Potenzierte Arzneimittel werden zum Beispiel in der homöopathischen und anthroposophischen Medizin eingesetzt. Das Herstellungsverfahren gemäß Homöopathischen Arzneibuch (HAB) umfasst die Prozesse des Verdünnens und anschließenden Schüttelns (bei Flüssigkeiten) oder Verreibens (bei Feststoffen), die zusammengefasst (d.h. Verdünnen plus Schütteln oder Verdünnen plus Verreiben) als Potenzierung bezeichnet werden.

Die FR-A-2 659 553 offenbart ein Verfahren zur Herstellung einer homöopathischen Zusammensetzung mit speziellen Verdünnungs - und Vermischungsschritten.

Dabei wird ein Teil der Ausgangssubstanz mit z. B. 9 Teilen des Potenziermediums (der Trägersubstanz) geschüttelt oder verrieben (potenziert). Üblich sind die Verdünnungsstufen 1:10 (D-Potenzen) und 1:100 (C-Potenzen). Von Ärzten, die homöopathische Arzneien einsetzen, wird bestätigt, dass auch und insbesondere Arzneien der Potenzstufen D30 (entspricht einer Verdünnung von 1:10³⁰) und höher (z. B. D200) wirksam sind. Dabei enthält die Trägersubstanz rechnerisch kein Molekül der Ausgangssubstanz mehr, da die Größenordnung der Avogadroschen Zahl (6,023x10²³) bei der Stufe D24 (oder entsprechend C12) überschritten wurde. Erklärt wird die dennoch auftretende Wirksamkeit in der Literatur damit, dass eine "Information" von der Ausgangssubstanz auf die Trägersubstanz übergehe. Welcher Art diese Information ist oder wie sie gespeichert wird, darüber gibt es keine gesicherten Erkenntnisse.

Wissenschaftlich ist die Therapie mit potenzierten Arzneien umstritten, da bisher keine allgemein akzeptierte theoretische Erklärung existiert. Auch ein Wirkungsprinzip, das mit der bekannten Physiologie und Biochemie verträglich ist, ist bisher nicht bekannt. Da seit der Entdeckung des Potenzierprinzips seit über 200 Jahren Ärzte und Patienten die therapeutische Wirksamkeit potenzierter Arzneien jedoch immer wieder bestätigen (auch als Tierarzneimittel), hat sich diese Arznei- und Therapieform trotz Erklärungsnotstandes, bis heute dennoch gehalten.

Die Herstellung der entsprechenden Arzneimittel ist im homöopathischen Arzneibuch (HAB) geregelt. Als Trägersubstanz (Potenziermedium) für potenzierte Arzneien sind Wasser, Alkohol (Äthylalkohol) und Milchzucker üblich. Flüssige Arzneien werden je nach Vorschrift mit Alkohol oder mit Wasser potenziert.

In Wirksamkeitsstudien mit potenzierten Arzneien gab es bisher keine eindeutigen Ergebnisse. Manche Studien belegen eine erhöhte Wirksamkeit gegenüber Placebo als Vergleich. Andere Studien konnten keine vom Placebo signifikant unterschiedliche Wirkung finden.

Daraus kann man schließen, dass Hersteller solcher Arzneien die Voraussetzungen für gute Wirksamkeit nicht vollständig kennen und nur mehr oder minder zufällig erreichen. Daraus folgt weiter, dass auch die Vorschriften des HAB, die für das Erreichen von Wirksamkeit erforderlichen Voraussetzungen nicht vollständig umfassen.

Hintergrund des eher zufälligen Erfolges ist die Tatsache, dass eben bisher unbekannt ist, wie die Trägersubstanz (das Potenziermedium) die Information der Ausgangssubstanz übernimmt und speichert und wie es zu einer Wirksamkeit solcherart potenzierter Arzneien kommen kann.

Natürliche Wässer und Leitungswässer enthalten immer Spuren von gebundenen Aminosäuren (proteinische Stoffe) und von freien Aminosäuren. Auch beim Umfüllen des Potenziermediums an der Luft werden zusätzlich geringe Mengen luftgetragener freier und gebundener Aminosäuren durch das Potenziermedium aufgenommen, wie wir bei entsprechenden Untersuchungen gefunden haben.

Die Alkoholherstellung erfolgt durch die Vergärung von Wein oder Obst und die anschließende Destillation (Brennen) zu Branntwein oder Obstschnaps. Ziel der Destillation ist neben der Abtrennung des wässrigen Anteils vom alkoholischen die Abtrennung gesundheitsschädlicher Anteile (z. B. Methylalkohol) vom Genussalkohol. Dabei können außer Äthylalkohol (Äthanol) zusätzlich weitere Stoffe wie z. B. andere Alkohole, Aldehyde, Ester sowie dampfflüchtige Proteine, Aminosäuren, Glykoproteine, Lipoproteine, Glykoside und Lipide in das Destillat übergehen. Diese zusätzlichen Stoffe sind von entscheidender Bedeutung für die Wirksamkeit potenzierter Arzneien. Dies ist bisher nicht bekannt. Deshalb werden Alkoholsorten eingesetzt, die diese Stoffe nicht oder nur in geringerem Maße enthalten.

Die heutigen Produktionsverfahren legen auf Reinheit der für die Arzneimittelherstellung eingesetzten Stoffe großen Wert. Darin wird ein Teil der Produktqualität gesehen. Dies kann ein weiterer Grund für die Verwendung von Alkoholqualitäten ohne die oben genannten Begleitstoffe sein. Mit steigendem Grad der "Reinigung" wird dabei jedoch unbewusst die Eignung für wirksame potenzierte Arzneien weiter herabgesetzt.

Beim Wasser wird ebenfalls auf Reinheit geachtet. So wird Wasser durch Mehrfachdestillation, Vollentsalzung, Ultrafiltration, Umkehrosmose und Bestrahlung mit ultraviolettem Licht (als Einzelverfahren oder in Kombination) besonders gereinigt, oder wenn bereits ausreichend reines Wasser zur Verfügung steht, dieses zumindest auf seine Reinheit kontrolliert. Durch Herstellung der potenzierten Arzneien unter Reinraumbedingungen wird auch der Anteil luftgetragener gebundener und freier Aminosäuren in der Luft gemindert. Entsprechend weniger kann in das Potenziermedium übergehen.

So summieren sich die Effekte heutiger Herstellungsverfahren bei potenzierten Arzneien und ihren Rohstoffen in Richtung der Verminderung der Wirksamkeit. Dies fällt jedoch kaum auf, da nicht alle Maßnahmen zugleich und schlagartig getroffen werden, sondern eine Maßnahme nach der anderen im Verlauf von Jahren und Jahrzehnten eingeführt und gesteigert eingeführt wurde. Da eine Wirksamkeitskontrolle potenzierter Arzneien im Labor bis jetzt nicht möglich ist, kann die nachlassende Wirkung auch nur in der therapeutischen Praxis auffallen. Derartige Verminderungen werden zwar von Praktikern immer wieder vermutet, lassen sich jedoch kaum prüfen.

Der Erfindung liegt das Problem zugrunde, ein Verfahren zur Herstellung eines potenzierten Arzneimittels zu entwickeln, durch das sich die Wirksamkeit des potenzierten Arzneimitteln im Vergleich zu den heute verwendeten potenzierten Arzneimitteln wesentlich erhöht.

Dieses Problem wird erfindungsgemäß dadurch gelöst, dass ein Potenziermedium mit einem gegenüber den derzeit verwendeten Potenziermedien erhöhten Gehalt an gebundenen Aminosäuren von über 400 nmol/l und/oder freien Aminosäuren von über 200 nmol/l verwendet wird.

Diese Lösung beruht auf eine Abkehr von der bisherigen Praxis bei der Herstellung von Potenziermedien. Es wurde wissenschaftliche Literatur daraufhin durchgesehen, welche Herstellungsbedingungen für homöopathische Präparate angegeben werden, wenn die Wirksamkeitsstudien (an Patientenkollektiven oder im Tierversuch) das Ergebnis "keine nachweisbare Wirksamkeit" lieferte. Dabei ergab sich, dass in solchen Fällen oft erwähnt wird, dass als Potenziermedium doppelt destilliertes Wasser oder besonders reiner Alkohol eingesetzt wurde.

Zusätzlich zur Erfahrung und Literaturauswertung gibt es eine weitere Begründung unserer Maßnahmen zur Steigerung der Wirksamkeit potenzierter Arzneien. Diese Begründung ist ein neues theoretisches Konzept, wie potenzierte Arzneien die Information speichern und wie sie wirken könnten. Dieses theoretische Konzept schließt an bekanntes biochemisches und physiologisches Wissen an und hilft schließlich auch, die Wirkungsweise potenzierter Arzneien zu verstehen.

Beim Verdünnen und Schütteln oder Verreiben (das man physikalisch als einen Anregungsvorgang ansehen kann) breitet sich die elektromagnetische Struktur der Moleküle der Ausgangssubstanz in ihrer molekularen Umgebung aus. Dies wirkt auf die im Potenziermedium enthaltenen proteinischen Substanzen und Aminosäuren und formt diese um in ein Abbild der Ausgangssubstanz. Es wird also angenommen, dass die im wässrigen Milieu besonders leicht beweglichen Aminosäuren und Peptidmoleküle sich unter dem Einfluss der Moleküle der Ausgangssubstanz umordnen. Sie nehmen vermutlich eine Struktur an, die der Struktur der Ausgangssubstanz (dem zu potenzierenden Arzneistoff) ähnelt.

Bildhaft kann man sich dies wie die Anfertigung eines Gipsabdruckes (Negativform) vorstellen. Im nächsten Schritt wird der Gipsabdruck wieder aufgefüllt und es entsteht eine positive Nachbildung des Originals. Im nächsten Schritt wird von dieser Nachbildung wieder ein Negativ-Abdruck entstehen, und so fort. Ein dem Wechsel von Positiv- und Negativ-Form entsprechender Wirkungswechsel ist in Laborexperimenten in der Literatur tatsächlich beschrieben worden. Allerdings blieb auch dabei unerklärt, worauf er zurückzuführen ist.

Die geschilderte Strukturweitergabe kann man als die stoffliche Grundlage der in der Literatur postulierten Informationsübertragung ansehen.

Der Potenziervorgang kann also darin bestehen, dass die im Potenziermedium enthaltenen ungeordneten Mengen proteinischer Stoffe ihre Struktur ändern, sich unter dem Einfluss der in überwiegend geordneter Weise vorhandenen Ausgangssubstanz in eine dieser ähnliche Gestalt umformen. Auch im nächsten Potenzierschritt kann man sich wieder einen vergleichbaren Vorgang vorstellen. Die geordneten proteinischen Stoffe bilden um sich ein gemeinsames Feld, das stärker ist, als die zwar zahlenmäßig zahlreicheren, jedoch wegen ihrer Verschiedenartigkeit nicht zu einer sich durchsetzenden Ordnung fähigen proteinischen Substanzen des Potenziermediums. So setzt beim Schütteln die geordnete Minderheit ihre Ordnung (Struktur, Gestalt) durch und prägt sie den anderen auf.

Ein ähnlicher Wechsel ist in der Bio-Chemie als Gen-Antigen-Prinzip bekannt. Auch sind zum Antigen wieder Anti-Antigene bekannt. Schon dieses Prinzip der biochemischen Ähnlichkeitsweitergabe kann auch theoretisch nahelegen, dass proteinische Stoffe am Potenzierprozess beteiligt sein können.

Die Strukturweitergabe kann möglich sein, weil proteinische Stoffe Kettenmoleküle oder Molekülkomplexe sind, die im Wasser oder Alkohol als bewegliche Struktur vorliegen. Diese Struktur wird durch Wasserstoffbrücken stabilisiert. Diese können jedoch bei entsprechender elektromagnetischer Anregung, wie sie beim Schütteln in den dipolaren Flüssigkeiten Wasser und Alkohol zwangsläufig vorliegt, umgeformt werden. Ähnlich kann man sich den Vorgang bei den proteinischen Substanzen und Aminosäuren im Milchzucker vorstellen, nur dass man hier die in den letzten 15 Jahren bekanntgewordenen Festkörper-Clusterstrukturen, zu Hilfe nehmen muss. Festkörper-Cluster sind Strukturen, die zwischen der Molekül- und Kristallstruktur liegen. Sie sind geordnet, jedoch nicht so unbeweglich wie Festkörpermoleküle oder Kristalle, sondern eher umordnungsfreudig.

Typische Molekülkomplexe der hier gemeinten Art sind z. B. Polyaminosäuren. Polyaminosäuren sind als Peptide, Proteine, Enzyme, Hormone, Eiweiße, Albumine usw. bekannt. Aber auch Zucker sind zur Bildung von komplizierten Strukturen geeignet und als Polysaccharide bekannt. Dies gilt gesteigert, wenn zusätzlich Bindungen an Peptide vorliegen (Glycoproteine). Ebenso sind fettige und ölige Verbindungen (Lipide) zu Verkettungen fähig, wiederum insbesondere in Verbindung mit Peptiden (Lypoproteine).

Eine weitere Möglichkeit von Molekülkomplexen der hier gemeinten Art sind lockere Verkettungen von freien Aminosäuren. Aminosäuren weisen ein saures und ein basisches Molekülende auf. In Proteinen liegt eine feste Verbindung (kovalente Bindung) vor (Peptidbindung). Ohne Peptidbindung ist eine lockere Anziehung zwischen basischen und sauren Enden verschiedener Aminosäuren möglich. Dies ist eine niederenergetische Bindung ähnlich der der Wasserstoffbrückenbindung zwischen verschiedenen Wassermolekülen. Aminosäuren existieren in großer Vielfalt; es sind über 300 Arten bekannt.

Auch Zuckermoleküle können sich schwach anziehen und Mikrostrükturen bilden. Clusterstrukturen bei Feststoffen sind in der Literatur beschrieben. Beim Milchzucker wird gemäß der Erfindung ebenfalls die Beteiligung von Proteinen oder/und Aminosäuren als entscheidend angesehen, die jedoch, je nach Herstellungsweg des Milchzuckers, unterschiedlich vertreten sind.

Der bislang nicht geklärte eigentliche Träger der Informationsspeicherung in potenzierten Arzneien wird also in den genannten strukturbildenden Substanzen gesehen. Die Klasse der proteinischen Substanzen ist nun gerade die zentrale Substanzgruppe der Biochemie. An der Mehrzahl der physiologischen Reaktionen sind proteinische Substanzen wie Enzyme, Hormone, Peptide usw. beteiligt. Das bedeutet, dass man vermuten darf, dass auch die potenzierten Arzneien auf diese Weise wirken. Dadurch, dass eine in gewisser Weise einseitige Einzelsubstanz (die Ausgangssubstanz) ihre Gestalt in eine Vielzahl anderer proteinischer Substanzen prägt, kann man sich diese sogar als flexibler im Erreichen aller Organe eines Menschen oder Tieres vorstellen, so dass sie wirksamer sein können, ohne die Einseitigkeit einer bestimmten chemischen Wirkung zu zeigen. Dies macht zugleich die Wirksamkeit und Nebenwirkungsarmut plausibel.

Die Erfindung beruht auf der Erkenntnis, dass gerade in den sogenannten "Verunreinigungen" von Wasser und Alkohol und Milchzucker gerade ihre bisher unerkannten Wirkstoffanteile liegen. Die Erfindung besteht deshalb darin, zur Wirksamkeitssteigerung Wässer, Alkohole und Milchzucker als Bestandteil potenzierter Arzneien einzusetzen, die proteinogene und physiologische, freie Aminosäuren und/oder proteinisches Material (gebundene Aminosäuren) enthalten oder ihnen solche Stoffe zuzusetzen.

Das nach dem erfindungsgemäßen Verfahren hergestellte, potenzierte Präparat kann auch dazu verwendet werden, auf Milchzucker (Lactose) in Tabletten-, Kugel- oder sonstiger Form versprüht oder damit vermengt, verrieben oder auf sonstige Weise vermischt zu werden, um ein potenziertes Arzneimittel mit anderer Darreichungsform herzustellen. Besonders vorteilhaft für die vorliegende Erfindung ist es, wenn als Potenziermedium ein Alkohol verwendet wird, der durch Gärung von Pflanzen gewonnen wurde, die zu einem besonders hohen Gehalt an freien und gebundenen Aminosäuren im Alkohol führen (z. B. Schlehe). Auch bei der Verwendung von Wasser als Potenziermedium kann man vorsehen, dass die freien und gebundenen Aminosäuren zunächst einem Alkohol entzogen und dann dem Wasser zugesetzt werden.

## Patentansprüche

1. Verfahren zur Herstellung eines potenzierten Arzneimittels für die Anwendung bei Menschen, Tieren oder Pflanzen, **dadurch gekennzeichnet, dass** ein Potenziermedium mit einem gegenüber den derzeit verwendeten Potenziermedien erhöhten Gehalt an gebundenen Aminosäuren von über 400 nmol/l und/oder freien Aminosäuren von über 200 nmol/l verwendet wird.

2. Verfahren nach Anspruch 1, **dadurch gekennzeichnet, dass** ein Potenziermittel mit einem Gehalt an gebundenen Aminosäuren von über 800 nmol/l und/oder einem Gehalt an freien Aminosäuren bei über 400 nmol/l verwendet wird.

3. Verfahren nach den Ansprüchen 1 oder 2, **dadurch gekennzeichnet, dass** ein Potenziermittel mit einem Gehalt an gebundenen Aminosäuren und/oder freien Aminosäuren von etwa dem dreifachen Wert heutiger Potenziermedien verwendet wird.

4. Verfahren nach zumindest einem der vorangehenden Ansprüche, **dadurch gekennzeichnet, dass** die gebundenen und/oder freien Aminosäuren dem Potenziermedium als Begleitstoffe zugesetzt werden.

5. Verfahren nach Anspruch 4, **dadurch gekennzeichnet, dass** die Begleitstoffe aus Früchten gewonnen werden.

6. Verfahren nach zumindest einem der vorangehenden Ansprüche, **dadurch gekennzeichnet, dass** die gebundenen und/oder freien Aminosäuren durch Beigeben von natürlich destilliertem Fruchtalkohol erzeugt werden.

7. Verfahren nach zumindest einem der vorangehenden Ansprüche, **dadurch gekennzeichnet, dass** als Potenziermittel ein Fruchtalkohol mit hohem Gehalt an Aminosäuren verwendet wird.

8. Verfahren nach Anspruch 7, **dadurch gekennzeichnet, dass** als Fruchtalkohol ein Alkohol aus Schlehen benutzt wird.

9. Verfahren nach zumindest einem der vorangehenden Ansprüche, **dadurch gekennzeichnet, dass** die freien und/oder gebundenen Aminosäuren durch Kontakt des Potenziermediums mit der natürlichen Luft zugesetzt werden.

10. Verfahren nach zumindest einem der vorangehenden Ansprüche, **dadurch gekennzeichnet, dass** als Potenziermittel Wasser verwendet wird.

11. Verfahren nach zumindest einem der vorangehenden Ansprüche, **dadurch gekennzeichnet, dass** als Potenziermittel Milchzucker verwendet wird.

## Claims

1. Method for the production of a potentiated medicament for use on humans, animals or plants, **characterized in that** a potentiating medium containing higher levels than currently used potentiating media of over 400 nmol/l of combined amino acids and/or over 200 nmol/l of free amino acids is used.

2. Method according to Claim 1, **characterized in that** a potentiating medium containing over 800 nmol/l of combined amino acids and/or over 400 nmol/l of free amino acids is used.

3. Method according to Claim 1 or Claim 2, **characterized in that** a potentiating medium containing some three times the level of current potentiating media of combined amino acids and/or free amino acids is used.

4. Method according to at least one of the preceding claims, **characterized in that** the combined and/or free amino acids are added to the potentiating medium as accompanying substances.

5. Method according to Claim 4, **characterized in that** the accompanying substances are derived from fruit.

6. Method according to at least one of the preceding claims, **characterized in that** the combined and/or free amino acids are produced by addition of naturally distilled fruit alcohol.

7. Method according to at least one of the preceding claims, **characterized in that** a fruit alcohol containing a high level of amino acids is used as potentiating medium.

8. Method according to Claim 7, **characterized in that** an alcohol derived from sloes is used as fruit alcohol.

9. Method according to at least one of the preceding claims, **characterized in that** the free and/or combined amino acids are added through contact of the potentiating medium with natural air.

10. Method according to at least one of the preceding claims, **characterized in that** water is used as potentiating medium.

11. Method according to at least one of the preceding claims, **characterized in that** lactose is used as potentiating medium.

## Revendications

1. Procédé pour la fabrication d'un médicament dynamisé pour utilisation sur les hommes, les animaux et les plantes, **caractérisé en ce qu'**on utilise un milieu de dynamisation présentant une teneur en acides aminés liés de plus de 400 nmol/l et/ou une teneur en acides aminés libres de plus de 200 nmol/l, plus élevées que celles des milieux de dynamisation actuellement utilisés.

2. Procédé selon la revendication 1, **caractérisé en ce qu'**on utilise un milieu de dynamisation présentant une teneur en acides aminés liés de plus de 800 nmol/l et/ou une teneur en acides aminés libres de plus de 400 nmol/l.

3. Procédé selon les revendications 1 ou 2, **caractérisé en ce qu'**on utilise un milieu de dynamisation présentant une teneur en acides aminés liés et/ou en acides aminés libres d'environ trois fois la valeur des milieux de dynamisation actuels.

4. Procédé selon au moins une des revendications précédentes, **caractérisé en ce que** les acides aminés liés et/ou libres sont ajoutés au milieu de dynamisation sous la forme de substances d'accompagnement.

5. Procédé selon la revendication 4, **caractérisé en ce que** les substances d'accompagnement sont obtenues à partir de fruits.

6. Procédé selon au moins une des revendications précédentes, **caractérisé en ce que** les acides aminés liés et/ou libres sont produits par addition d'alcool de fruit distillé naturellement.

7. Procédé selon au moins une des revendications précédentes, **caractérisé en ce qu'**on utilise comme milieu de dynamisation un alcool de fruit présentant une teneur élevée en acides aminés.

8. Procédé selon la revendication 7, **caractérisé en ce qu'**on utilise comme alcool de fruit un alcool de prunelle.

9. Procédé selon au moins une des revendications précédentes, **caractérisé en ce que** les acides aminés libres et/ou liés sont ajoutés par contact du milieu de dynamisation avec l'air naturel.

10. Procédé selon au moins une des revendications précédentes, **caractérisé en ce que** le milieu de dynamisation utilisé est de l'eau.

11. Procédé selon au moins une des revendications précédentes, **caractérisé en ce que** le milieu de dynamisation utilisé est du sucre de lait.
